# EUROPÄISCHE PATENTANMELDUNG

(11) **EP 1 400 261 A1**
(43) Veröffentlichungstag der Anmeldung: **24.03.2004**
(21) Anmeldenummer: 02020517.5
(22) Anmeldetag: 13.09.2002
(51) Int. Cl.: A61N 2/02

(54) **Verfahren zur Planung der Stimulation hyper/hypometabolischer Kortexareale**

(71) Anmelder: BrainLAB AG, 85551 Kirchheim/Heimstetten (DE)
(72) Erfinder: Hartlep, Andreas, Dr., 80803 München (DE); Tanner, Philipp, Dr., 80769 München (DE); Eichhammer, Peter, Dr., 93133 Burglengenfeld (DE); Langguth, Berthold, Dr., 93049 Regensburg (DE)
(74) Vertreter: Schwabe - Sandmair - Marx

(57) **Zusammenfassung**

Die Erfindung betrifft ein Verfahren zur Planung der Stimulation hyper/hypometabolischer Kortexareale, bei dem
- mittels eines bildgebenden Verfahrens Patientenanatomiedaten ermittelt werden,
- die Position der hyper/hypometabolischen Kortexareale in der Patientenanatomie sowie die Position einer Stimulationseinrichtung mittels eines medizinischen Navigationssystems erfasst werden,
- die Position der hyper/hypometabolischen Kortexareale gegenüber der Position der Stimulationseinrichtung registriert bzw. referenziert wird, und bei dem
- auf der Basis der relativen Positionsinformationen die optimierte Positionierung der Stimulationseinrichtung geplant wird.

Ferner betrifft sie ein Programm, das, wenn es auf einem Computer läuft oder in einem Computer geladen ist, den Computer veranlasst, ein solches Verfahren durchzuführen, und ein Computerprogramm-Speichermedium, das ein solches Programm aufweist.

## Beschreibung

Die vorliegende Erfindung betrifft die Planung der Stimulation hyper/hypometabolischer Kortexareale. Insbesondere betrifft sie die Planung der Stimulation, bevorzugt magnetischen Stimulation, hypermetabolischer Kortexareale, die der Manifestierung des systemischen Tinnitus zugeordnet sind.

Die magnetische Stimulation kortikaler Bereiche ist vor allem aus dem sogenannten "Brain-Mapping" bekannt, wobei direkt oder indirekt ein Kartierung von Hirnfunktionen erfolgt, um die Auffindung bestimmter funktioneller Himareale zu ermöglichen.

Aus der US 5,738,625 ist ein Verfahren bekannt, um Nervenzellen magnetisch zu stimulieren. Die US 5,644,234 beschreibt ein Kemspinresonanz(MR)-Verfahren, wobei die Position einer Mikrospule in einem Objekt ermittelt werden soll. Aus der WO 98/06342 sind ein Verfahren und eine Vorrichtung zur transcranialen magnetischen Stimulation des Gehirns bekannt, wobei ein etwa halbkugelförmiger von Spulen umwickelter Magnetkern zum Erzeugen eines Stimulationssignals verwendet wird. Bei der beschriebenen Vorrichtung und dem Verfahren soll die Sprachfunktion lokalisiert werden.

Die US 6,394,969 beschreibt ein Tinnitus-Masking und eine Surpressor-Vorrichtung zur Verwendung gepulsten Ultraschalls. Die US 6,366,813 beschreibt eine Vorrichtung und ein Verfahren für die intracraniale Stimulation zur optimalen Kontrolle neurologischer Krankheiten. Die US 6,402,678 beschreibt eine Einrichtung und ein Verfahren zur Behandlung von Migräne. Die US 6,425,852 beschreibt eine Vorrichtung und ein Verfahren zur transcranialen magnetischen Himstimulation, einschließlich der Behandlung von Depressionen und der Lokalisierung und Charakterisierung von Sprachschwierigkeiten.

Die US 6,258,032 beschreibt ein Verfahren zur Diagnose und Behandlung von Vasospasmus-Symptomen. Die US 6,132,361 beschreibt eine transcraniale Himstimulation. Die US 5,769,778 beschreibt eine magnetische, nicht-konvulsive Stimulationstherapie. Die US 5,061,234 beschreibt einen magnetischen Neural-Stimulator für die Neurophysiologie. Die DE 39 37 793 A1 beschreibt eine Vorrichtung zur induktiven Stimulation von erregbarem Gewebe. Die DE 44 08 110 A1 beschreibt ein Verfahren und eine Vorrichtung zur neuromagnetischen Stimulation. Die DE 199 14 762 A1 beschreibt eine Spulenanordnung zur transcranialen magnetischen Stimulation. Die DE 199 52 191 C1 beschreibt eine Vorrichtung zur Magnetstimulation von Neuronen und/oder Nervenfasern mit einer Spule und eine Verwendung von Mitteln zur Kühlung der Spule.

Es ist die Aufgabe der vorliegenden Erfindung, ein Verfahren zur Planung der Stimulation hyper/hypometabolischer Kortexareale vorzuschlagen, bei dem sichergestellt ist, dass die Stimulationseinrichtung optimal positioniert wird. Insbesondere ist eine Aufgabe der vorliegenden Erfindung, ein solches Planungsverfahren bereitzustellen, das sich für die Stimulation, insbesondere die magnetische Stimulation hypermetabolischer Kortexareale eignet, die der Manifestierung des systemischen Tinnitus zugeordnet sind.

Erfindungsgemäß wird diese Aufgabe gelöst durch ein Verfahren zur Planung der Stimulation hyper/hypometabolischer Kortexareale, bei dem mittels eines bildgebenden Verfahrens Patientenanatomiedaten ermittelt werden, die Position der hyper/hypometabolischen Kortexareale in der Patientenanatomie sowie die Position einer Stimulationseinrichtung mittels eines medizinischen Navigationssystems erfasst werden, die Position der hyper/hypometabolischen Kortexareale gegenüber der Position der Stimulationseinrichtung registriert bzw. referenziert wird, und bei dem auf der Basis der relativen Positionsinformationen die optimierte Positionierung der Stimulationseinrichtung geplant wird.

Durch die Einbindung des medizinischen Navigationssystem in die Planung der Stimulation der hyper/hypometabolischen Kortexareale wird eine optimierte Planung zur Verfügung gestellt, welche sicherstellt, dass die gewünschten kortikalen Bereiche mit hoher Treffgenauigkeit stimuliert werden, wodurch es erstmals auch möglich wird, die Stimulation solcher Kortexareale zu planen, die der Manifestierung des systemischen Tinnitus zugeordnet sind, wie dies gemäß einer bevorzugten Ausführungsform der vorliegenden Erfindung vorgesehen ist.

In speziellerer Ausgestaltung betrifft die Erfindung ein Verfahren, bei dem bei der Ermittlung der Patientenanatomiedaten einerseits funktionelle Anatomiedaten, durch funktionale Bilderfassungsverfahren ermittelt werden, insbesondere durch funktionelle Magnetresonanzerfassung und/oder Positronen-Emissions-Tomographie (PET), und andererseits strukturelle, im Navigationssystem positionell zuordnungsfähige Anatomiedaten durch konventionelle Bilderfassungsverfahren ermittelt werden, insbesondere durch Magnetresonanzerfassung bzw. Kernspintomographie, wobei dann die Navigations-Registrierung bzw. -Referenzierung für die funktionalen Anatomiedaten durch ein computerunterstütztes Matchingverfahren, insbesondere ein graphisches Matchingverfahren, mit den strukturellen Anatomiedaten erfolgt, so dass die funktionalen Anatomiedaten für die Navigation zur Verfügung stehen. Mit einer solchen Ausführungsvariante ist die Zuordnung solcher funktionellen Anatomiedaten und struktureller Anatomiedaten möglich bzw. deren Integration im Rahmen dieser Planung, um eine für den Behandlungserfolg optimale Navigation bzw. ein optimales Tracking zu gewährleisten.

Das mit der Erfindung verwendete Navigationssystem kann eines sein, welches auf der optischen Erfassung aktiv oder passiv abstrahlender Markeranordnungen basiert, die am Patienten, insbesondere an dessen Kopf und an der Stimulationseinrichtung angeordnet werden. Stattdessen oder zusätzlich hierzu kann ein Navigationssystem verwendet werden, welches auf der magnetischen bzw. induktiven Erfassung positionsgebender Einrichtungen, insbesondere Spulen bzw. Schwingkreise, basiert, die am Patienten, insbesondere an dessen Kopf und an der Stimulationseinrichtung angeordnet werden.

Die Stimulationseinrichtung kann eine Kortex-Stimulationsspule sein.

Es ist von Vorteil, die erfassten Navigationsdaten auf einer Bildausgabe zusammen mit den Planungsergebnissen auszugeben.

In weiterer vorteilhafter Ausgestaltung wird die Stimulationseinrichtung bzw. die Stimulationsspule im Rahmen der Planung kalibriert. Ferner kann mit Vorteil für die Stimulationseinrichtung bzw. die Stimulationsspule im Rahmen der Planung eine computergestützte Feldverteilungssimulation erfolgen, mit Hilfe der die Stimulationsbereiche bestimmt werden.

Die Erfindung betrifft ferner ein Programm, das, wenn es auf einem Computer läuft oder in einen Computer geladen ist, den Computer veranlasst, ein Verfahren durchzuführen, wie es oben beschrieben wurde. Außerdem betrifft die Erfindung ein Computerprogramm-Speichermedium, das ein solches Programm aufweist.

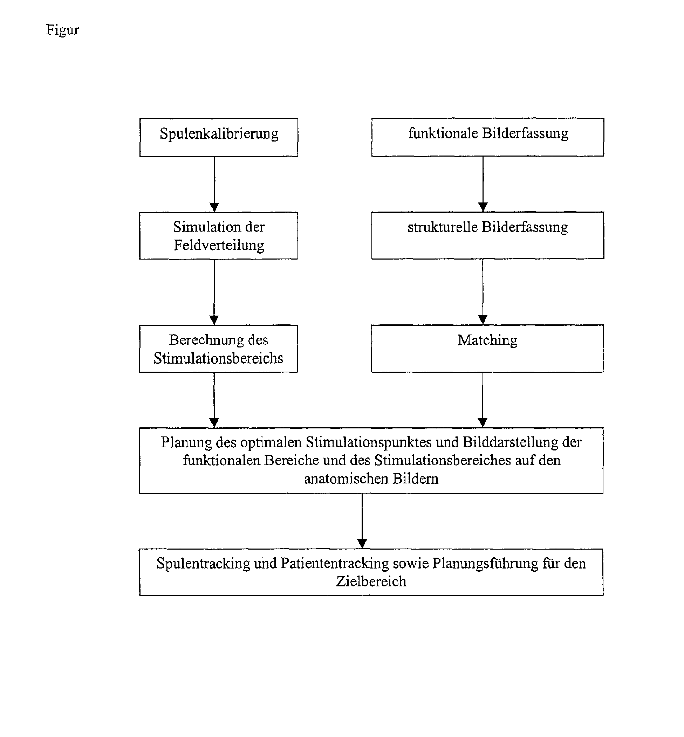

Die Erfindung wird im Weiteren anhand der beiliegenden Figur erläutert, welche als Blockdiagramm einen Ablauf des erfindungsgemäßen Planungsverfahrens aufzeigt.

Gemäß dem im Weiteren beschriebenen Ausführungsbeispiel wird die erfindungsgemäße Planung für die Tinnitus-Behandlung unter Verwendung der navigierten, transcranialen magnetischen Stimulation eingesetzt. In der ersten Phase laufen zwei Prozessteile unabhängig voneinander und möglicherweise parallel ab. Im ersten Prozessteil erfolgt die Vorbereitung der Stimulationsspule, durch die ein Magnetfeld erzeugt wird, welches bei der Behandlung die hyper/hypometabolischen Kortexareale stimulieren wird. Dabei erfolgt zunächst eine Kalibrierung der Spule und danach eine Simulation (Computersimulation) der Feldverteilung. Diese Feldverteilung wird dabei recht aufwändig schon so simuliert, wie sie sich tatsächlich im Bereich der Gehirnstruktur ergeben wird, wobei das magnetische Feld einer Spule hier natürlich sehr viel komplizierter aufgebaut ist als ohne "störende" Gehirnstruktur im Feldaufbau. Nachdem nun bekannt ist, wie die Feldverteilung aussehen wird, lässt sich auch berechnen, wo der von der Spule ausgehende Stimulationsbereich relativ zur Spulenposition sein wird. Die Spule wird, um sie positionell verfolgen zu können, mit Navigations-Markierungen (optisch, magnetisch) versehen, und sie kann damit in einem Navigationssystem getrackt werden. Dadurch können auch die vorher simulierten bzw. berechneten Simulationsbereiche getrackt werden, deren Positionsverhältnis zur Spule wie oben aufgeführt vorab ermittelt wurde.

Separat von obiger Prozessabfolge, möglicherweise zeitlich parallel, wird von einem Tinnitus-Patienten eine Bilderfassung durchgeführt. Diese Bilderfassung besteht einerseits aus der funktionalen Bilderfassung, bei der mittels funktionaler Magnetresonanzerfassung oder Positronen-Emissions-Tomographie bzw. anderer geeigneter Verfahren festgestellt wird, wo sich die funktionalen Kortexbereiche, die stimuliert werden sollen, bei diesem Patienten befinden. Im vorliegenden Fall, wo der primäre und sekundäre Hör-Kortex aufgefunden werden sollen, wird der Patient zum Beispiel Schallreizen ausgesetzt, und über die funktionale Bilderfassung wird bestimmt, welcher Kortexbereich dabei reagiert. Diese funktionalen Bilder werden also verwendet, um den Bereich der hypermetabolischen Aktivitäten im Gehirn zu bestimmen, welcher der Manifestierung des systemischen Tinnitus zugeordnet ist.

Ferner werden unabhängig hiervon Strukturbilder des Patienten erstellt, beispielsweise durch Kernspintomographie, CT oder sonstige geeignete Verfahren. Diese strukturelle Bilderfassung kann in bekannter Weise durch den Einsatz von Markern am Kopf des Patienten so in ein Navigationssystem eingeordnet werden, dass die Positionen der Gehimstruktur gegenüber den Markern und damit im Navigationssystem verfügbar werden. Im weiteren Schritt, nämlich dem Schritt des Matching werden die erzeugten Strukturbilder gegenüber den Funktionsbildern registriert, um die räumliche Position bestimmter funktionaler Kortexareale verfügbar zu machen. Dabei kann ein graphisches, computergestütztes Matching-Verfahren verwendet werden, das die Daten der erfassten Anatomiebereiche beispielsweise anhand von in den Bildern erkennbaren Strukturabgrenzungen zuordnet.

Aus diesem Teil des Prozessablaufes erhält man demnach nach dem Matching einen Patienten-Anatomiedatensatz, mit dem bzw. auf dem navigiert werden kann und der die funktionalen Bereiche aufzeigt. Dieser Bilddatensatz wird nun verwendet, um die vorbereitete Spule zu navigieren und damit wird eine Planung des optimalen Stimulationspunktes sowie eine Bilddarstellung der funktionalen Bereiche und des Stimulationsbereiches auf den anatomischen Bildern ermöglicht. In dem kombinierten Stimulations-Neuronavigationssystem werden über eine Bilddarstellung, beispielsweise einen Bildschirm diejenigen Bereiche gezeigt, die stimuliert werden sollten, um einen Tinnitus zu behandeln. Das Navigationssystem ist dazu in der Lage, die Raumposition der Spule und des Kopfes zu erfassen und die tatsächliche und geplante Position maximaler Stimulierung zu detektieren und aufzuzeigen, um einen behandelnden Arzt zum richtigen Stimulationsgebiet (Zielbereich) hinzuführen.

## Patentansprüche

1. Verfahren zur Planung der Stimulation hyper/hypometabolischer Kortexareale, bei dem
- mittels eines bildgebenden Verfahrens Patientenanatomiedaten ermittelt werden,
- die Position der hyper/hypometabolischen Kortexareale in der Patientenanatomie sowie die Position einer Stimulationseinrichtung mittels eines medizinischen Navigationssystems erfasst werden,
- die Position der hyper/hypometabolischen Kortexareale gegenüber der Position der Stimulatiohseinrichtung registriert bzw. referenziert wird, und bei dem
- auf der Basis der relativen Positionsinformationen die optimierte Positionierung der Stimulationseinrichtung geplant wird.

2. Verfahren nach Anspruch 1, bei dem die Stimulation hypermetabolischer Kortexareale geplant wird, die der Manifestierung des systemischen Tinnitus zugeordnet sind.

3. Verfahren nach Anspruch 1 oder 2, bei dem, bei der Ermittlung der Patientenanatomiedaten einerseits funktionelle Anatomiedaten, durch funktionale Bilderfassungsverfahren ermittelt werden, insbesondere durch funktionelle Magnetresonanzerfassung und/oder Positronen-Emissions-Tomographie (PET), und andererseits strukturelle, im Navigationssystem positionell zuordnungsfähige Anatomiedaten durch konventionelle Bilderfassungsverfahren ermittelt werden, insbesondere durch Magnetresonanzerfassung bzw. Kernspintomographie, wobei dann die Navigations-Registrierung bzw. -Referenzierung für die funktionalen Anatomiedaten durch ein computerunterstütztes Matching-Verfahren, insbesondere ein graphisches Matching-Verfahren, mit den strukturellen Anatomiedaten erfolgt, so dass die funktionalen Anatomiedaten für die Navigation zur Verfügung stehen.

4. Verfahren nach einem der Ansprüche 1 bis 3, bei dem ein Navigationssystem verwendet wird, welches auf der optischen Erfassung aktiv oder passiv abstrahlender Markeranordnungen basiert, die am Patienten, insbesondere an dessen Kopf und an der Stimulationseinrichtung angeordnet werden.

5. Verfahren nach einem der Ansprüche 1 bis 4, bei dem ein Navigationssystem verwendet wird, welches auf der magnetischen bzw. induktiven Erfassung positionsgebender Einrichtungen, insbesondere Spulen bzw. Schwingkreise, basiert, die am Patienten, insbesondere an dessen Kopf, und an der Stimulationseinrichtung angeordnet werden.

6. Verfahren nach einem der Anspruche 1 bis 5, bei dem als Stimulationseinrichtung eine Kortex-Stimulationsspule verwendet wird.

7. Verfahren nach einem der Ansprüche 1 bis 6, bei dem die erfassten Navigationsdaten auf einer Bildausgabe zusammen mit den Planungsergebnissen ausgegeben werden.

8. Verfahren nach einem der Ansprüche 1 bis 7, bei dem die Stimulationseinrichtung bzw. die Stimulationsspule im Rahmen der Planung kalibriert wird.

9. Verfahren nach einem der Ansprüche 1 bis 8, bei dem für die Stimulationseinrichtung bzw. die Stimulationsspule im Rahmen der Planung eine computergestützte Feldverteilungssimulation erfolgt, mit Hilfe der die Stimulationsbereiche bestimmt werden.

10. Programm, das, wenn es auf einem Computer läuft oder in einem Computer geladen ist, den Computer veranlasst, ein Verfahren gemäß einem der Ansprüche 1 bis 9 durchzuführen.

11. Computerprogramm-Speichermedium, das ein Programm nach Anspruch 10 aufweist.
